# EUROPEAN PATENT APPLICATION

(11) **EP 2 835 134 A1**
(43) Date of publication of application: **11.02.2015**
(21) Application number: 14183739.3
(22) Date of filing: 15.09.2011
(51) Int. Cl.: A61K 36/06, A61P 39/06, A23L 1/30, C12N 1/14, C12N 3/00, A61K 36/062

(54) **Fungal extracts and uses thereof**

(30) Priority: 16.09.2010 FI 20105950
(62) Divisional of application: 11824639.6
(71) Applicant: Metsäntutkimuslaitos, 01370 Vantaa (FI); Cermedi Oy, 33560 Tampere (FI)
(72) Inventor: Uusitalo, Hannu, 33014 TAMPEREEN YLIOPISTO (FI); Sarjala, Tytti, 39700 Parkano (FI); Viitala, Katja, 60100 Seinäjoki (FI); Potila, Hannamaria, 39700 Parkano (FI); Huhtala, Anne, 33340 Tampere (FI)
(74) Representative: Kolster Oy Ab

(57) **Abstract**

The present invention relates to compositions and methods of inhibiting, treating or preventing age related or other retinal diseases, neurodegenerative diseases, or heart and vascular diseases. The invention is in the field of dietary (foods and food supplements), nutraceutical and pharmaceutical compositions containing extracts from root-associated fungal species, or single or synergistically functioning multiple compounds therefrom.

## Description

### FIELD OF THE INVENTION

The present invention relates to methods of inhibiting, treating or preventing age related or other macular degeneration, neurodegenerative diseases, or heart and vascular diseases. The invention is in the field of dietary (foods and food supplements), nutraceutical and pharmaceutical compositions containing extracts and single or synergistically functioning multiple compounds of the extracts from root-associated fungal species including but not limited to *Acephala sp., Articulospora sp., Dermea sp., Ascomycete* clones, *Meliniomyces sp., Phialocephala sp., Phialocephala sp., Phialophora sp., Umbelopsis* sp., and Pezizomycotina.

### BACKGROUND OF THE INVENTION

Reactive oxygen species, e.g. in the form of free radicals and peroxides, are normal intermediates of cellular metabolism but may cause toxic effects and damage all components of cells, including proteins, lipids, and DNA, in situations where the cell's ability to detoxify the reactive intermediates or repair the resulting damage is imbalanced. Oxidative stress is known to be involved in many human diseases including atherosclerosis, Parkinson's disease, Alzheimer's disease, heart failure, myocardial infarction and degenerative retinal diseases.

Age-related macular degeneration (AMD) is the leading cause of blindness in the elderly worldwide. It is characterized by a progressive loss of vision due to the degenerative and neovascular changes in the central region of retina, macula. World Health Organization (WHO) Global Eye Disease Survey conservatively indicates that 50 million persons suffer from AMD and 14 million persons are blind or severely visually impaired because of AMD. AMD has a severe impact on the physical and mental health of the aging population, not only for the patients themselves but also for their families and it is becoming a major and increasing public health burden. Direct medical costs of AMD care is estimated approximately $2.8 billion per year in the US and about $2.6 billion per year in Australia. The global costs of AMD are 101.1 million euros in UK, 60.5 million euros in Italy, 91.4 million euros in Germany and 51.3 million euros in France. Without an effective treatment, the number of patients severely disabled by AMD is expected to increase in the next 20 years by more than 50%. The prevalence of early AMD in the age-category 65-74 years is 15%, in the age-category 75-84 years 25%, and in persons 85 years and older 30%. A reasonable overall estimate of the prevalence of late AMD in persons aged 65-74 years is 1%, increasing to 5% in persons aged 75-84 years, and up to 13% in persons 85 years and older. For example, in Pirkanmaa district the absolute number of persons over 65 is estimated to increase by 75% by year 2030. To avoid the serious and expensive consequences of this scenario attempts should be made to better understand the pathogenesis of these diseases, to prevent their development and progression and finally to develop cost effective means to treat and replace the destroyed structure and function of the retina.

The etiology of AMD is known to be multifactorial, i.e., in addition to a strong genetic component, environmental risk factors such as aging, smoking, obesity, hypertension and hypercholesterolemia may predispose to AMD. Chronic oxidative stress and inflammation are strongly linked to AMD pathogenesis. AMD is generally divided in non-exudative (dry, atrophic) and exudative (wet) forms.

A hallmark of an early AMD is the detrimental accumulation of lysosomal lipofuscin in the retinal pigment epithelial cells (RPE) and the presence of extracellular drusen deposits between the basal lamina of the RPE and the inner collagenous layer of Bruch's membrane. High amounts of lipofuscin and drusens predict the progression of AMD. In response to chronic oxidative stress and inflammation, choroidal neovascularization may be developed in certain (10-15%) cases leading to the wet form of AMD. In the exudative form of AMD, new vessels sprout from the choroidal capillaries through Bruch's membrane into the sub-RPE space or into retinal layers.

Given the severity and vast socio-economical impact, there is an identified need in the art for the development of new therapeutics for oxidative stress -related diseases, like AMD, Alzheimers's disease and many other chronic neurodegenerative diseases.

Mushrooms are a non-taxonomic group of fungi having a distinctive fruiting body large enough to be seen with the naked eye and to be picked by hand. Use of mushrooms and mushroom extracts for therapeutic and medicinal purposes has long traditions especially in folk medicine. Mushrooms are an interesting target in modern medical research for identifying novel bioactive substances and pharmaceuticals. For example, cholesterol drug lovastatin may be isolated from certain mushrooms such as *Pleurotus os-treatus.*

All plants in natural ecosystems appear to be symbiotic with fungi. The establishment and growth of most plants require, or is enhanced, by the presence of specialized fungi in the soil. There are several different types of associations between the plant roots and fungi and a vast majority of terrestrial plant species form mycorrhizal associations. In the case of coniferous trees such as Scots pine (*Pinus sylvestris*) they are called ectomycorrhizas (ECM), due to the characteristic structure of the plant-fungus relationship. The ECM is characterised by a hyphal mantle around the symbiotic root tip, external fungal mycelium on the surface and fungal network between the cortex cells of the plant roots (called Hartig net). The highly diverse group of fungi, which live in a mutualistic or symbiotic relationship, can have profound impacts on plant communities through increasing fitness by conferring abiotic and biotic stress tolerance, increasing biomass and decreasing water consumption.

A relatively unstudied group of root-associated fungi that reside in the tissues of living plants (intercellularly or intracellularly) are called endophytes. Unlike mycorrhizal fungi, the fungal endophytes of the roots lack extraradical (outside the root) hyphal networks and mantles (sheaths around the roots). Only a few of these plants have ever been completely studied relative to their endophytic biology, but it has been shown that fungal isolates from a single grass plant root system from a single location may reveal 49 different phylotypes and only seven of them were closely similar to known sequences. Fungal endophytes have been found in all woody plants studied to date. Trees and shrubs usually contain numerous fungal species; however, there has been little attempt to correlate ecological and evolutionary aspects of fungal endophytes of grasses versus woody plants.

The dark septate endophytes (DSE) comprise a heterogeneous assemblage of fungi that inhabits the roots of plant species worldwide. DSE are a miscellaneous group of ascomycetous anamorphic fungi that colonize root tissues intracellularly and intercellularly. DSE have been defined as conidial or sterile ascomycetous fungi that colonize living plant roots without causing apparent negative effects such as tissue disorganization. However, also fungi typically forming ectendomycorrhizas of conifers may also be included into this group. These associations are characterized with a thin mantle and Hartig net including penetration into intracellular space, which is not typical to ECM. DSE are a diverse group of fungi and may include a number of fungi forming ectendomycorrhizas. It has been proposed that DSE symbioses, like mycorrhizas, are multifunctional and not limited to nutritional acquisition and host growth response. Molecular data indicate the diverse origin of DSE and taken together, all available observations indicate DSE to be a poorly defined group of fungi referring liberally whenever melanized, septate hyphae are observed colonizing roots either intercellularly or intracellularly.

Fungal species definitions have been based on overall phenotypic similarity, ecological distributions, reproductive isolation, evolutionary divergence, and combinations of the above. In practice, fungal species concepts have evolved from strictly morphological descriptions, to biological (reproductive) species concepts, to phylogenetically (deoxyribonucleic acid, DNA, divergence) based species concepts.

At present, majority of therapeutically useful fungal substances have been derived from the fruiting bodies of mushrooms. Given the diversity of the Kingdom Fungi, there is a great potential in identifying novel bioactive or pharmaceutical compositions from other fungal groups such as root-associated fungi.

### BRIEF DESCRIPTION OF THE INVENTION

The present invention relates to extracts of root-associated fungi and to the use of said extracts or components thereof as antioxidants, especially in protecting retinal pigment epithelium cells against oxidative stress.

An object of the present invention is to provide extracts of one or more root-associated fungi. In some embodiments, said fungus is derived from a Scots pine. In other embodiments said fungus is selected from a group consisting of *Acephala* sp. *(e.g. Acephala applanata), Articulospora sp., Dermea* sp., *Ascomycete* clones, *Meliniomyces sp. (e.g. Meliniomyces variabilis), Phialocephala* sp. *(e.g. Phialocephala fortinii), , Phialophora* sp. *(e.g. Phialophora lignicola, i.e. Lecythophora lignicola), Umbelopsis* sp., and Pezizomycotina derived from roots of a Scots pine or from those of other woody plants.

Another object of the present invention is to provide a process for producing fungal extract described herein. Such a process may comprise the steps of a) providing a root sample of a woody plant, b) surface sterilizing said sample, c) transferring said sample to an isolation medium for culturing root-associated fungus contained in said sample, d) optionally breaking down said fungal culture, and e) extracting said fungal culture by pressing or with a suitable solvent.

Still another object of the present invention is to provide fungal extracts obtainable by the process described above.

A further object of the present invention is a medical use of the extracts or components thereof described herein. Thus, in some embodiments the extracts or components thereof are provided as medicaments. In further embodiments, said extracts or components thereof are provided for treating oxidative stress -related diseases including retinal diseases like age-related macular degeneration and neurodegenerative disorders.

The above object may be formulated differently, i.e. as a method of preventing, treating, ameliorating and/or alleviating an oxidative stress -related disease in a human subject in need thereof. Said method comprises administering to said patient a therapeutically efficient amount of a fungal extract or one or more components thereof described herein.

Furthermore, the present invention provides pharmaceutical and nutritional compositions comprising fungal extracts described herein or components thereof.

Further specific embodiments of the invention are set forth in the dependent claims.

Other embodiments, objects, details and advantages of the present invention will become apparent from the following drawings, detailed description and examples.

### BRIEF DESCRIPTION OF THE DRAWINGS

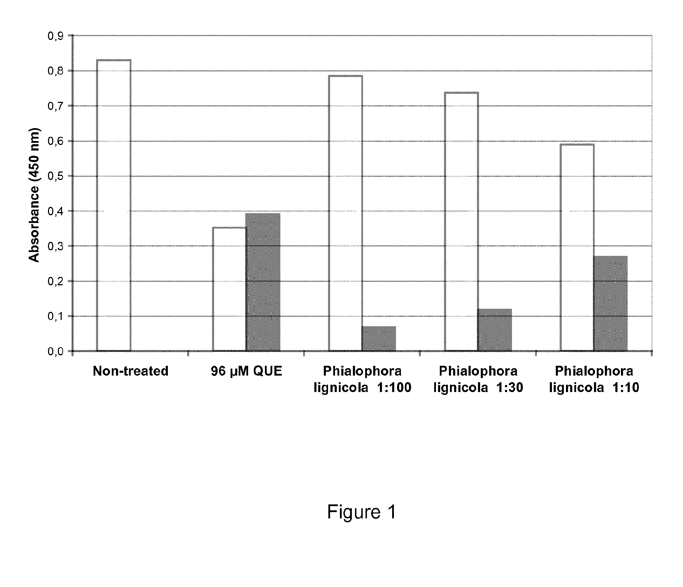

In the following the invention will be described in greater detail by means of preferred embodiments with reference to the attached drawings, in which
Figure 1 is a graph showing the results of a WST-1 cytotoxicity test of human retinal pigment epithelial cells (hRPE) after exposure to oxidative stress. Open bars represent non-exposed cells, whereas black bars represent cells exposed to hydrogen peroxide. Extracts of *Phialophora lignicola* protected the cells against oxidative damage in a dose dependent manner. Quercetin (QUE), a well known plant-derived antioxidant, was used as a positive control. Non-treated cells did not survive after exposure to hydrogen peroxide -induced oxidative stress.

### DETAILED DESCRIPTION OF THE INVENTION

The present invention is based on a surprising finding that extracts of root-associated fungi are efficient as antioxidants, and have a protective effect on human retinal pigment epithelial cells (hRPE cells) against oxidative stress.

The term "root-associated fungus" as used herein refers to a fungus that resides in association with plant roots. The term includes ectomycorrhizal fungi characterized by a hyphal mantle around the symbiotic root tip, external fungal mycelium on the surface and fungal network between the cortex cells of the plant roots, as well as endophytes residing in the root tissues of living plants intercellularly or intracellularly.

In connection with the present invention, roots of Scots pine trees were collected from the peatland forest and cleaned from soil for isolation of the fungi. Identification of the fungal species grown on pure cultures was done with molecular methods by amplifying fungal DNA (ITS1F and ITS2 or ITS4 primers) with PCR (Polymerase Chain Reaction) followed by nucleotide sequencing (White et al. 1990, Gardes and Bruns, 1993, Korkama et al. 2007).

Identified root-associated fungal species included *Acephala* sp. *(e.g. Acephala applanata), Articulospora sp., Dermea sp., Ascomycete* clones, *Meliniomyces* sp. *(e.g. Meliniomyces variabilis), Phialocephala* sp. *(e.g. Phialocephala fortinii), , Phialophora* sp. *(e.g. Phialophora lignicola, i.e. Lecythophora lignicola), Umbelopsis* sp., and Pezizomycotina.

In some embodiments of the present invention, the root-associated fungus may be derived from a plant other than Scots pine including genera such as *Betula, Cornus, Aralia, Rubus.* Accordingly, the root-associated fungal species suitable for use in various aspects and embodiments of the present invention are limited neither to those strains purified from the roots of Scots pine nor to the fungal taxonomic groups mentioned above.

The term "fungal culture" as used herein means a culture representing one or more fungal species, which may be produced by any suitable method including, but not limiting to, sequential translocation of the target fungal mycelium on growth medium to isolate a single fungal strain from a surface sterilized root material of woody plants, such as Scots pine, as a pure culture.

The term "culturing conditions" as used herein means any suitable media, which may be used for cultivating the fungi, and any suitable container, growing temperature, time, or other factors, which may have effect on the fungal growth and the compound production of the fungi. The culturing media include, but are not limited to, modified Melin-Norkrans (MMN2) medium (Marx 1969) with or without agar, or on modified Hagem medium (Modess 1941) with or without agar.

The term "extract" as used herein means a substance obtained by any extraction method, whether or not the crude extract has been fractioned or purified by usual methods such as chromatography or filtration. Extraction methods include, but are not limited to pressing and solvent extraction, and any suitable solvent may be used. The solvent may be an organic or inorganic solvent, or a mixture of suitable solvents. Typically, deionized water or a phosphate balanced buffer (PBS), pH 7.4 is used as an inorganic solvent, and an aqueous ethanol mix is used as the organic solvent. However, also other inorganic and organic solvents may be used. The extraction solvent may also be a supercritical fluid such as supercritical water or CO₂. The term fungal extract includes those obtained from fungal cultures of single or multiple fungal species or strains, as well as mixtures of said extracts.

Accordingly, fungal extracts according to the present embodiments may be prepared from cultivated fungal mycelium under conditions suitable for fungal growth as defined above. Typical culture time varies between two to twelve weeks, and typical culture temperature varies between +18-+25 °C. It is desirable to break down the fungus tissue prior to obtaining the fungal extract. The fungus tissue may be broken down by physical treatments such as grinding with liquid nitrogen or any other suitable method preserving the chemical composition of the fungal material as intact as possible. In this way, the extraction can be carried out efficiently. However, tissue breaking procedure may be avoided by using larger amounts of fungal material and/or high temperature and/or high pressure during the following extraction. The extraction is carried out by pressing or with a suitable solvent defined above. If desired, the extraction may be repeated one or more times under the same or different conditions.

The fungal extracts may be concentrated and/or purified for further use. Concentration can be carried out by conventional techniques such as vacuum drying or freeze drying. The extract material may be purified to yield a purified extract and also to separate single compounds of the extract using standard techniques such as liquid-liquid extraction, column chromatography, fractional distillation, preparative TLC (thin layer chromatography), preparative HPLC (high performance liquid chromatography), CPC (centrifugal partition chromatography) or other techniques known to those skilled in the art.

As used herein, the term "components of the fungal extract", and any equivalents thereof, refers to one or more isolated and/or purified compounds derived from said extract. The term also refers to synthetic counterparts of the compounds found in said extracts as well as to said compounds produced in genetically modified organisms other than the original fungi.

The present fungal extracts show antioxidant activity. As used herein, the term "antioxidant" refers to substances having an ability to slow or prevent the oxidation of other molecules. Methods and assays for determining the antioxidant activity of a given substance are well known in the art.

Accordingly, the present fungal extracts or components thereof may be used to treat, prevent, inhibit, ameliorate or alleviate various oxidative stress-related disorders. Such disorders include, but are not limited to degenerative retinal conditions such as age-related macular degeneration and neurodegenerative disorders such as Alzheimer's disease, Parkinson's disease, and Huntington's disease.

In connection with the present invention it was shown that the fungal extracts disclosed herein are efficient in compensating for the deactivating effect of hydrogen peroxide on human retinal pigment epithelial cells (hRPE). In particular, extracts obtained from *Phialophora lignicola* were efficient in protecting the cells against oxidative damage. It is noteworthy that the compensatory effect was dose-dependent and that not all antioxidant substances showed a similar effect on hRPE cells. For instance, secoisolariciresinol (SECO), a known antioxidant found in many softwood species, e.g. *Picea, Abies* or *Pinus,* did not protect hRPE cells from oxidative damage. Moreover, an extract of *Paxillus involutus,* a mushroom commonly known as the brown roll-rim, common roll-rim, or poison pax, showed superior antioxidant activity in an assay for total phenolics according to the Folin-Ciocalteu as well as in a FRAP assay testing ferric reducing/antioxidant power, but failed to protect hRPE cells against oxidative stress.

Thus, in one particular embodiment, the present fungal extracts or components thereof may be used to treat, prevent, inhibit, ameliorate and/or alleviate degenerative retinal conditions, especially age-related forms of macular degeneration, by administering a therapeutically effective amount of the extract or one or more components thereof to a patient in need of such treatment, prevention, inhibition amelioration and/or alleviation. The term "therapeutically effective amount" as used herein refers to a dose required to confer a beneficial effect in a patient with a condition or disease in which treatment is sought. A therapeutically efficient amount may vary, as recognized to persons skilled in the art, for instance depending on the route and time of administration, and physiological characteristics including age, gender and general health of the patient to be treated.

The above aspects may be formulated in an alternative way, i.e. such that some embodiments of the present invention provide the present fungal extracts or components thereof as antioxidant substances for preventing, treating, inhibiting or ameliorating and/or alleviating oxidative stress - related conditions or diseases, such as neurodegenerative disorders and degenerative retinal conditions, especially age-related forms of macular degeneration. Accordingly, the fungal extracts or components thereof may be used for the manufacture of a medicament for said oxidative stress -related conditions.

In one aspect the invention provides a composition comprising a fungal extract, or components thereof, of various embodiments of the invention. The composition may be a pharmaceutical composition, in which case the extract or one or more components thereof is in admixture with one or more pharmaceutically acceptable carriers or excipients. The composition may also be a nutritional composition in the form of food or beverage.

Pharmaceutical compositions may take the form of, for example, tablets, capsules, caplets or similar dosage forms prepared by conventional means with pharmaceutically acceptable carriers and/or excipients such as binding agents, fillers, lubricants, disintegrants, or wetting agents. The extract or one or more components thereof may also be incorporated into liquids, solutions, or syrups, or formulated as dispersions or suspensions by dissolving a dried extract or one or more pure compounds thereof. Such liquid compositions may take the form of, for example, a drink, tincture or drop.

In some embodiments, the pharmaceutical composition is formulated as an ophthalmic formulation for application to the eye. In further embodiments, the pharmaceutical composition takes the form of a topical ophthalmic formulation. Such formulations may comprise ophthalmologically acceptable preservatives, surfactants, viscosity enhancers, gelling agents, tonicity enhancers, penetration enhancers, buffers, and the like.

It will be obvious to a person skilled in the art that, as the technology advances, the inventive concept can be implemented in various ways. The invention and its embodiments are not limited to the examples described above but may vary within the scope of the claims.

### Example 1. Isolation of root-associated fungi and preparation of fungal extracts

Roots of Scots pine trees were collected from the peatland forest and cleaned from soil for isolation of the fungi. Root surface sterilization was performed by dipping the root segments briefly (about 15 sec) in 70% ethanol, followed by washing with sterile H₂O, soaking in 30 % hydrogen peroxide for 1 min 30 sec, and washing with sterile water. Sterilized root tips were transferred to isolation medium (2% water agar, glucose + Streptomycin or Hagemagar+ Streptomycin).

Identification of the fungal isolates was done by amplifying fungal specific ITS-region with PCR using ITS1 F-ITS2 primer pair or ITS1F-ITS4 primer pair. These primer pairs have been designed by Gardes and Bruns (1993) and by White et al. (1990). High Pure PCR Product Purification Kit (Roche, Mannheim, Germany) was used to purify amplified DNA and direct sequencing of the purified PCR samples was performed with CEQ 8000 DNA analysis system and Quick Start Kit (Beckman Coulter Inc., Fullerton, California; USA). Sequences were compared to the information in GenBank to identify the fungal species.

One of the identified fungal strains was *Phialophora lignicola.* This fungus was maintained on MMN2 medium with agar, or on modified Hagem medium with agar. Other identified fungal strains are listed above in the detailed description of the invention.

The MMN2 liquid medium contained KH₂PO₄ (3.7 mM), (NH₄)₂HPO₄ (1.9 mM), CaCl₂•2H₂O (0.45 mM), NaCl (0.43 mM), MgSO₄•7H2O (0.61 mM), 100 µg thiamine HCl (0.3 µM), FeCl₃•6H₂O (18.5 µM), and glucose (41.6 mM) in deionized water, pH 5.7.

The modified Hagem medium contained KH₂PO₄ (3.7 mM), NH₄Cl (9.3 mM), MgSO₄•7H₂O (2.0 mM), thiamine HCl (0.15 mM), FeCl₃•6H₂O (18.5 µM), 5.0 g malt extract per liter, and glucose (27.8 mM) in deionized water, pH 5.0.

Agar (15 g/l) was used for solidification of the culture media for some fungal species. In such cases, a moist cellophane membrane (P 400, Visella Oy, Valkeakoski, Finland) was used on the agar medium to prevent the fungus to penetrate into the agar medium. One to three plugs, each 5 mm in diameter and cut from the margin of a 1-month-old fungal colony were then placed on the cellophane membrane and cultivated in the dark at room temperature. For other fungal species, fluidic medium on Petri dishes (25 or 80 ml per dish) or in bottles (150 ml per bottle) was used. Culturing bottles were stirred 50-100 rpm for aeration.

The fungal mass was collected after growth of four weeks and stored refrigerated at -20 °C until careful grinding in mortar with liquid nitrogen. The grinded fungus was divided into aliquots in polypropylene test tubes, weighed, and refrigerated at -20 °C before further processing. One aliquot for each fungal sample was weighed on a plastic Petri dish and dried at +45 °C to determine the percentage of moisture of the samples.

The extraction of the fungal mass was performed using three alternative solvents. One extractant used was a common phosphate buffered saline (PBS) of pH 7.4. One litre of PBS contained 8.0 g NaCl, 0.2 g KCI, 1.42 g Na₂HPO₄•2H₂O, and 0.23 g KH₂PO₄ in deionized water. Another alternative extractant used was deionized water and the third was 70% ethanol mixed in deionized water. Simultaneously, as the fungal samples were extracted, control samples in which the fungal material is replaced with deionized water were processed respectively, too.

For PBS (or water) extraction, the fungal samples were first vortexed rigorously for 1-2 minutes with 5 ml of PBS (or deionized water) per 1-3 g of fungal mass in sealed test tubes. Thereafter, the extraction tubes were incubated in a water bath of +95-+100 °C for 15 minutes, cooled in an ice-water bath, and vortexed rigorously for 1-2 minutes. In the end, the tubes were centrifuged 7000 x g for 10 minutes at +4 °C.

For ethanol extraction, the fungal samples were first vortexed rigorously for 1-2 minutes with 5 ml of 70 % ethanol per 1-3 g of fungal mass in sealed test tubes. Then the tubes were rotated in room temperature for 30 minutes, vortexed again and centrifuged 7000 x g for 10 minutes at +4 °C.

The supernatants of the extractions were poured off the fungal pellets before next extraction cycle. There were 2-3 extraction cycles for each sample. The supernatants (2-3 x 5 ml) were combined, mixed thoroughly and centrifuged again as before. The final supernatants were filtrated with a nylon syringe filter (diameter 25 mm, pore size of 0.2 µm). A known volume (e.g. 10 ml) from each filtrate was dried using a vacuum centrifuge at +25-+45 °C and dissolved thereafter in Dulbecco's Modified Eagle Medium DMEM/F-12 (Ham) 1X (1:1, Gibco). The rest of the filtrated supernatants were divided into aliquots and refrigerated at -20 °C (or below).

The samples dissolved in DMEM/F-12 were sterilized in a laminar flow chamber with PES syringe filters (diameter 25 mm, pore size 0.1 µm). Thereafter, these filtrates were used for bioactivity tests including, but not limited to, the cell model of age related macular degeneration.

### Example 2. Protection of human retinoplastic endothelium cells against oxidative stress by extracts of root-associated fungi

Human ARPE-19 retinal pigment epithelial cells (RPE) were obtained from American Type Culture Collection (USA). The cells were grown to confluency in a standard incubator in appropriate Dulbecco's MEM/Nut MIX F-12 medium (Gibco, UK). Oxidative stress was applied to the cells (1 x 10⁴ cells/well) by using various concentrations of H₂O₂. Cytotoxicity was evaluated by two cytotoxicity tests, WST-1 test. WST-1 test reagent was purchased from Roche, Basel, Switzerland. WST-1 test is based on the cleavage of the tetrazolium salt WST-1 (4-*3-(4-iodophenyl)-2-(4-nitrophenyl)-2H-5-tetrazolio*-1,3-benzene disulfonate, slightly red) to formazan (dark red) by various mitochondrial dehydrogenase enzymes. The mean optical density values corresponding to the non-treated controls were taken as 100%. The results were expressed as percentages of the optical density of treated vs. untreated controls. Dose-response curves were drawn from the results expressed as mean ± standard error of mean (SEM) by GraphPad Prism software (GraphPadTM, San Diego, US) using non-linear regression analysis. The EC₅₀ values, the concentrations of H₂O₂ and tested extracts (prepared as described in Example 1) that decreased the WST-1 reduction values to 50% of the controls, were determined when possible from the dose-response curves. The statistical significance of the differences between the cultures exposed to H₂O₂ and tested extracts without or with serum was determined with the Student's two-tailed t-test (GraphPad Prism). Differences were considered significant when P<0.05.

Extracts of *Phialophora lignicola* protected hRPE cells against oxidative stress statistically significantly. Figure 1 demonstrates that the level of protection was dose dependent.

### LIST OF REFERENCES

Gardes, M. and Bruns, T.D., 1993. ITS primers with enhanced specificity for basidiomycetes--application to the identification of my-corrhizae snd rusts. Molecular Ecology 2, 113-118.

Firuzi O, Lacanna A, Petrucci R, Marrosu G, Saso L (2005) Evaluation of the antioxidant activity of flavonoids by "ferric reducing power" assay and cyclic voltammetry. Biochim. Biophys. Acta 1721: 174-184.

Marx DH (1969) The influence of ectotrophic mycorrhizal fungi on the resistance of pine roots to pathogenic infections. I. Antagonism of mycorrhizal fungi to root pathogenic fungi and soil bacteria. Phytopathology 59: 159-163.

Modess O (1941) Zur Kenntniss der Mycorrhiza bildner von Kiefer und Fichte. Symbolae Botanicae Upsaliensis 5: 1-147.

Singleton VL, Orthofer R, Lamuela-Raventós RM (1999) Analysis of total phenols and other oxidation substrates and antioxidants by means of Folin-Ciocalteu reagent. Methods in Enzymology 299: 152-178.

Korkama, T., Fritze, H., Pakkanen, A., Pennanen, T., 2007. Interactions between extraradical ectomycorrhizal mycelia, microbes associated with the mycelia and growth rate of Norway spruce (Picea abies) clones. New Phytologist 173, 798-807.

White, T.J., Bruns, T., Lee, S., Taylor, J., 1990. Amplification and direct sequencing of fungal ribosomal RNA genes for phylogenetics. In: Innis, M.A., Gelfand, D.H., Sninsky, J.J., White, T.J. (Ed.), PCR protocols - a guide to methods and applications. Academic press, inc., pp. 315-322.

Wilson, B.J. Addy, H.D. Tsuneda, A. Hambleton, S., R.S. Currah, R.S. 2004. Phialocephala sphaeroides sp. nov., a new species among the dark septate endophytes from a boreal wetland in Canada. Can. J. Bot. 82: 607-617.

## Claims

1. An extract of a root-associated fungus or an isolated component thereof for use as a medicament, wherein said root-associated fungus is *Phialophora* sp, i.e. *Lecytophora* sp.

2. The extract or isolated component thereof according to claim 1, wherein said root-associated fungus is *Phialophora,* i.e. *Lecytophora.*

3. The extract or isolated component thereof according to claim 1 or 2, wherein said root-associated fungus is *Phialophora lignicola,* i.e. *Lecytophora lignicola.*

4. The extract or isolated component thereof according to any one of claims 1-3, wherein said root associated fungus has been derived from a Scots pine.

5. The extract or isolated component thereof according to any one of claims 1-4 for use in treating oxidative stress -related diseases selected from a group consisting of age-related macular degeneration and neurodegenerative disorders.

6. A pharmaceutical composition comprising a fungal extract or an isolated component thereof according to any of claims 1-4, or a fungal extract or an isolated component thereof for use according claim 5, and a pharmaceutically acceptable carrier.

7. A nutritional composition comprising a fungal extract or an isolated component thereof according to any of claims 1-4.
